# EUROPEAN PATENT APPLICATION

(11) **EP 1 609 795 A1**
(43) Date of publication of application: **28.12.2005**
(21) Application number: 04014911.4
(22) Date of filing: 25.06.2004
(51) Int. Cl.: C07F 9/50, C07F 15/00, B01J 31/24, C07C 43/225

(54) **Process for the preparation of asymmetrically substituted biaryldiphosphines**

(71) Applicant: Lonza AG, 4052 Basel (CH)
(72) Inventor: The designation of the inventor has not yet been filed

(57) **Abstract**

Provided is a process for the preparation of asymmetrically substituted biaryldiphosphine ligands of the formula 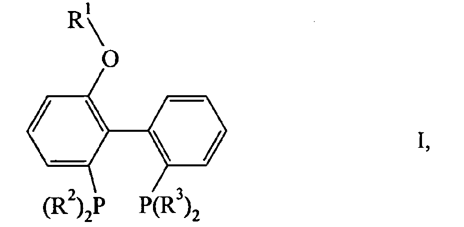 wherein R¹ is C₁₋₆-alkyl or C₃₋₁₀-cycloalkyl optionally being substituted with one or more halogen atoms, and
R² and R³ are independently selected from the group consisting of aryl, C₅₋₁₀-cycloalkyl and C₁₋₆-alkyl, wherein each aryl moiety is optionally substituted with one or more substituents selected from the group consisting of halogen atoms, nitro, amino, C₁₋₆-alkyl, C₁₋₆-alkoxy and di-C₁₋₆-alkylamino groups, and each C₁₋₆-alkyl, C₁₋₆-alkoxy, di-C₁₋₆-alkylamino and C₅₋₁₀-cycloalkyl group in R² and R³ optionally being substituted with one or more halogen atoms, from 2,2',6,6'-tetrabromobiphenyl by a sequence of halogen-metal exchanges and subsequent reactions.

## Description

Preparation of enantiomerically pure compounds is important to improve the effect of pharmaceutically active compounds and to restrict unwanted side effects of the "wrong" isomers. The invention relates to a process for the preparation of asymmetrically substituted biaryldiphosphine ligands and transition metal complexes thereof for the hydrogenation of unsaturated prochiral compounds using said complexes.

Asymmetric catalytic hydrogenation is one of the most efficient and convenient methods for preparing a wide range of enantiomerically pure compounds. Providing methods for the precise control of molecular chirality of pharmaceutical active compounds and compounds thereof tends to play an increasingly important role in synthetic chemistry. Several diphosphine ligand families are commonly known with their trade names, for example BINAP, CHIRAPHOS, DIOP, DUPHOS, SEGPHOS and TUNAPHOS.

Methods for the preparation of biaryldiphosphine ligands of the BINAP, SEGPHOS and TUNAPHOS families are disclosed in EP-A-025 663, EP-A-850945 and WO-A-01/21625, respectively. Furthermore WO-A-03/029259 discloses a synthesis of a fluorine derivative of SEGPHOS and its use.

In Pai, C.-C. et al., *Tetrahedron Lett.* **2002,** *43*, 2789-2792 the use of methylenedioxo and ethylenedioxo substituted biaryldiphosphine ligands for the asymmetric hydrogenation of ethyl 4-chloro-3-oxobutyrate is described. Further examples for the preparation of biaryldiphosphines and asymmetric hydrogenation reactions using catalysts derived from biaryldiphosphine ligands are disclosed in EP-A-0 926 152, EP-A-0 945 457 and EP-A-0 955 303. Usually both symmetrically and unsymmetrically substituted biaryldiphosphines are claimed, though only examples of symmetrically substituted ligands are disclosed. With only few specific exceptions, no general applicable synthetic route to unsymmetrically substituted biaryldiphosphines and catalysts derived therefrom is disclosed.

Biaryl diphosphine ligands consist of three different moieties, a rigid biaryl core, substituents to hinder biaryl rotation and usually two phosphine groups with voluminous substituents to complex a transition metal. Known examples of ligand systems have symmetric substitution patterns of the core and identical phosphine groups. As a rare example WO-A-02/40492 discloses asymmetric hydrogenation of ethyl 4-chloro-3-oxobutyrate, using a catalyst containing the ligand (*S*)-6-methoxy-5',6'-benzo-2,2'-bis(diphenylphosphino)-biphenyl. The (*S*)-alcohol is obtained with an enantiomeric excess (ee) of 83%.

EP-A-0 647648 and WO-A-02/40492 claim diphosphines with asymmetrically substituted biaryl core, but the disclosed synthetic principles are not suitable to produce a broad variety of different asymmetrically substituted biaryldiphosphine ligands.

For the synthesis of the inventive asymmetric biaryldiphosphines a major obstacle had to be overcome as depicted in Scheme 1. Any 2'-diphenylphosphino-2-lithiobiphenyl generated as an intermediate failed to yield an asymmetrically substituted biaryldiphosphine by condensation with a second chlorodiorganylphosphine component, if a single fluorine, chlorine or bromine atom or a single methoxy or dimethylamino group was attached to the 6-position (Miyamoto, T.K. et al., *J. Organomet. Chem.* **1989,** 373, 8-12; Desponds, O., Schlosser, M., *J. Organomet. Chem.* **1996,** 507, 257). The compounds undergo nucleophilic substitution at the phosphorus atom and cyclization to afford 1*H*-benzo[b]phosphindole (9-phosphafluorene). Known unsuccessful approaches to the inventive ligands are depicted in Scheme 1 below.

Here and hereinbelow the term "enantiomerically pure compound" comprises optically active compounds with an enantiomeric excess (ee) of at least 90 %.

Here and hereinbelow the term "C₁₋ₙ-alkyl" represents a linear or branched alkyl group having 1 to n carbon atoms. C₁₋₆-alkyl represents for example methyl, ethyl, propyl, isopropyl, butyl, isobutyl, *sec*-butyl, *tert*-butyl, pentyl and hexyl.

Here and hereinbelow the term "C₁₋ₙ-alkoxy" represents a linear or branched alkoxy group having 1 to n carbon atoms. C₁₋₆-alkoxy represents for example methoxy, ethoxy, propoxy, isopropoxy, butoxy, isobutoxy, *sec*-butoxy, tert-butoxy, pentyloxy and hexyloxy.

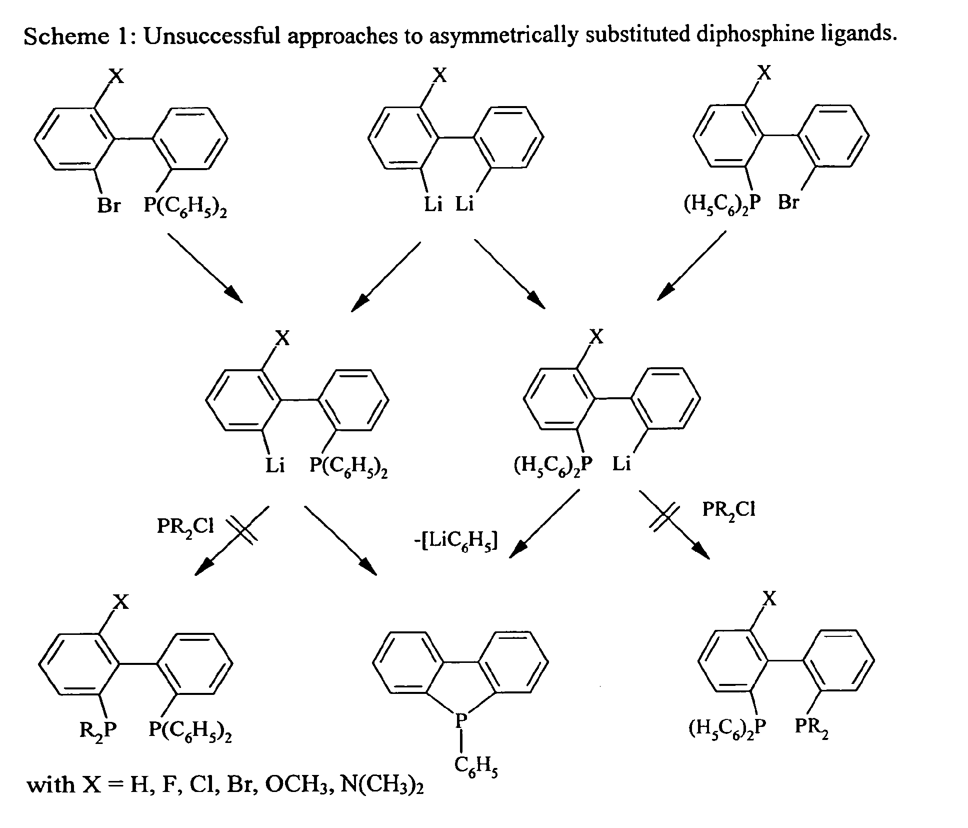

Here and hereinbelow the term "C₃₋ₙ-cycloalkyl" represents a cycloaliphatic group having 3 to n carbon atoms. C₅₋₁₀-cycloalkyl represents mono- and polycyclic ring systems such as cyclopentyl, cyclohexyl, cycloheptyl, cyclooctyl, adamantyl or norbomyl.

Here and hereinbelow the term "C₃₋ₙ-cycloalkoxy" represents a cycloalkoxy group having 3 to n carbon atoms. C₅₋₁₀-cycloalkyl represents for example cyclopentyloxy, cyclohexyloxy, cycloheptyloxy, cyclooctyloxy or cyclodecyloxy.

Here and hereinbelow the term "di-C₁₋₆-alkylamino" represents a dialkylamino group comprising two alkyl moieties independently having 1 to 6 carbon atoms. Di-C₁₋₆-alkylamino represents for example *N,N*-dimethylamino, *N,N*-diethylamino, *N*-ethyl-*N*-methylamino, *N*-methyl-*N*-propylamino, *N*-ethyl-*N*-hexylamino or *N,N*-dihexylamino.

Here and hereinbelow the term "aryl" represents an aromatic group, preferably phenyl or naphthyl optionally being further substituted with one or more halogen atoms, nitro and/or amino groups, and/or optionally substituted C₁₋₆-alkyl, C₁₋₆-alkoxy or di-C₁₋₆-alkylamino groups.

Here and hereinbelow the term "C₁₋₃-alcohols" represents methanol, ethanol, propanol and isopropanol.

Here and hereinbelow the term "C₁₋₃-alkanoic acids" represents formic acid, acetic acid and propanoic acid.

Considering the high stereocontrol and efficient action of enzymes, i.e. natural catalysts, great effort is spent to improve selectivity and efficiency of artificial catalysts, particularly for the production of pharmaceutically interesting compounds.

The technical problem to be solved by the present invention was to provide a method for the tailored synthesis of a series of biaryldiphosphines. A further problem to be solved was to establish said process in a robust manner to provide suitable amounts of ligands for the pharmaceutical industry. Furthermore, the general concept should start with an easily available compound and should contain few reaction steps, allowing the synthesis of a wide variety of ligands, only depending on the reaction sequence.

The problem could be solved according to the process of claim 1.

Provided is a process for the preparation of asymmetrically substituted biaryldiphosphine ligands of the formula 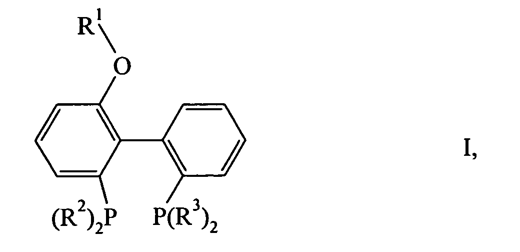 wherein R¹ is C₁₋₆-alkyl or C₃₋₁₀-cycloalkyl optionally substituted with one or more halogen atoms, and
R² and R³ are independently selected from the group consisting of aryl, C₅₋₁₀-cycloalkyl and C₁₋₆-alkyl, wherein each aryl moiety is optionally substituted with one or more substituents selected from the group consisting of halogen atoms, nitro, amino, C₁₋₆-alkyl, C₁₋₆-alkoxy and di-C₁₋₆-alkylamino groups, and each C₁₋₆-alkyl, C₁₋₆-alkoxy, di-C₁₋₆-alkylamino and C₅₋₁₀-cycloalkyl group in R² and R³ optionally being substituted with one or more halogen atoms,
comprising a first reaction sequence, wherein one bromine atom of 2,2',6,6'-tetrabromo-biphenyl 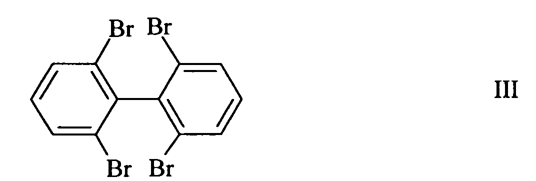 is exchanged with OR¹ by halogen-metal exchange and subsequent metal-hydroxy exchange, followed by an alkylation, to afford a compound of formula 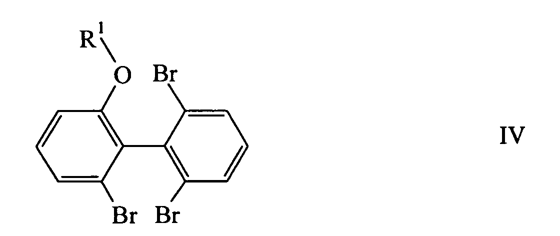 wherein R¹ is as defined above,
and a second reaction sequence, wherein one bromine atom of each aryl moiety of the compound of formula IV is exchanged with iodine by halogen-metal exchange and subsequent metal-iodine exchange, to afford a compound of formula 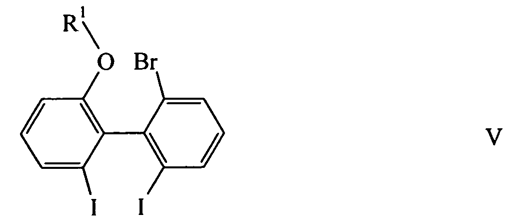 and further reaction sequences, wherein each reaction sequence comprises at least one halogen-metal exchange and subsequent metal-substituent exchange with the respective substituent, thereby exchanging the respective halogen atoms with substituents selected from the group consisting of hydrogen, diarylphosphino, di-C₁₋₆-alkylphosphino and di-C₅₋₁₀-cycloalkylphosphino.

The bromine-metal exchanges mentioned in the instant invention may be carried out with the required amount of the respective organometallic compound at a temperature below -40 °C ("low temperature bromine-metal exchange") or at a temperature of at last 0 °C ("high temperature bromine-metal exchange").

Chiral biaryldiphosphine ligands comprising a biaryl skeleton which is permanently twisted around the central carbon-carbon bond have two atropisomers. Asymmetric hydrogenation with transition metal complexes are preferably performed with one of the atropisomeres and optionally further chiral auxiliaries. Therefore, it should be appreciated that any reference to ligands of formula 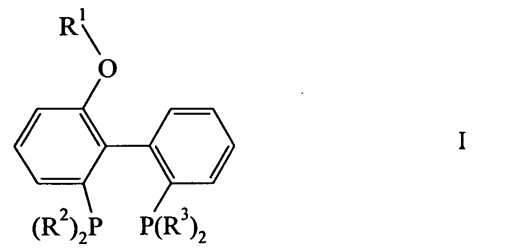 wherein R¹, R² and R³ are as defined above, implicitly includes its atropisomers 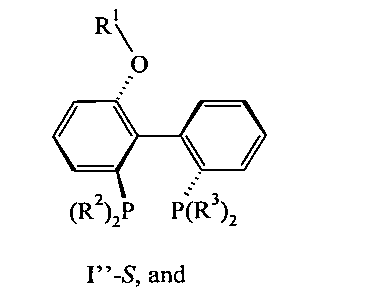 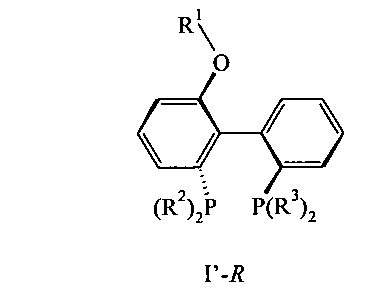 if not otherwise specified, e.g. by indicating their positive (+) or negative (-) optical rotation.

The undesired ring closure mentioned in Scheme 1 above can surprisingly be avoided by the inventive process. The reaction sequences of the present process for the preparation of compounds of formula I are depicted in Scheme 2. 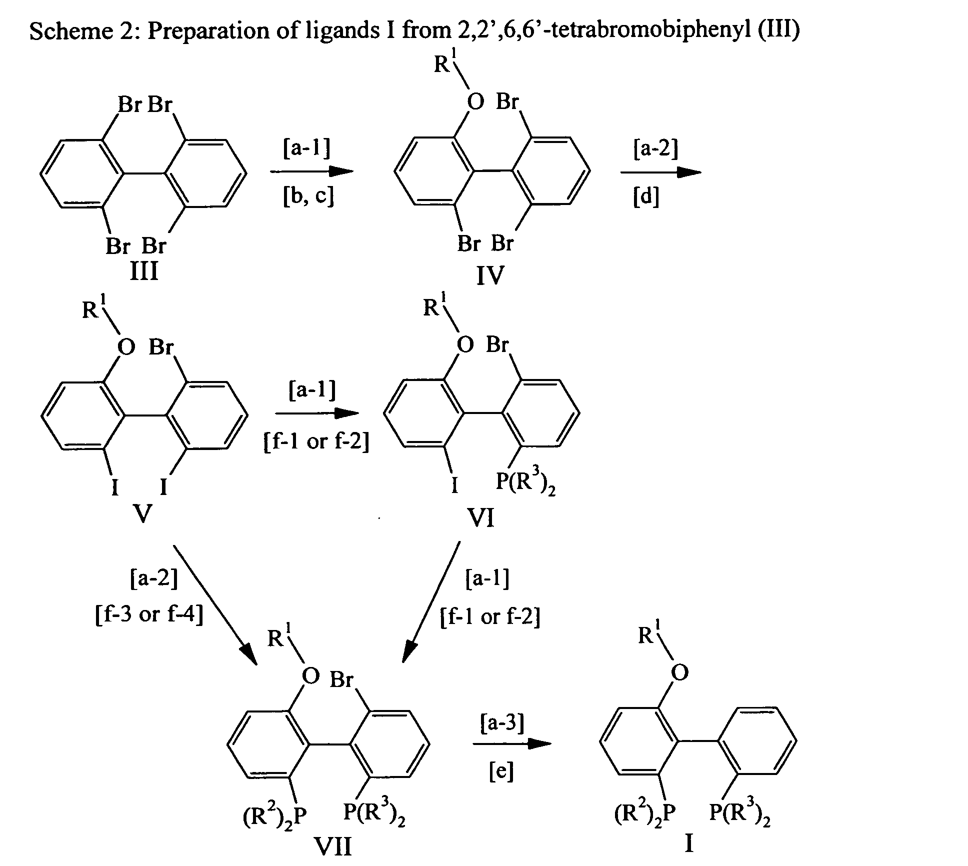 R¹, R² and R³ as described therein,
[a-1] = 1 eq. low temperature halogen-metal exchange;
[a-2] = 2 eq. low temperature halogen-metal exchange;
[a-3] = 1 to 2 eq. high temperature halogen-metal exchange;
[b] = borane oxidation
[c] = alkylation;
[d] = 2 eq. metal-iodine exchange;
[e] = hydrogen quenching;
[f-1] = 1 eq. metal-alkyl- or cycloalkylphosphine exchange;
[f-2] = 1 eq. metal-arylphosphine exchange;
[f-3] = 2 eq. metal-alkyl- or cycloalkylphosphine exchange;
[f-4] = 2 eq. metal-arylphosphine exchange.

The synthetic approach of Scheme 2 starts with 2,2',6,6'-tetrabromo-1,1'-biphenyl (III), wherein one bromine atom of III is replaced by an alkoxy or cycloalkoxy group. Compound III can be obtained by condensation of 1,3-dibromo-2-iodobenzene (II) according to Rajca A. et al., *J. Am. Chem. Soc.* **1996**, *118*, 7272-7279.

In a preferred embodiment, the first halogen-metal exchange of the compound of formula III is carried out with one equivalent of *n*-butyllithium at a temperature below -40 °C ("1 eq. low temperature halogen-metal exchange") in a polar solvent to afford a metallated intermediate. The following metal-hydroxy exchange is carried out by reacting the metallated intermediate with a borane or organoborate, followed by reaction with a peroxy compound in the presence of an alkali and/or earth alkali hydroxide, and the alkylation is carried out with an alkylating agent in the presence of a base.

Preferably the borane or organoborate is fluoromethoxyborane ethyl ether adduct, triiso-propylborate or trimethylborate. Particularly preferred the borane or organoborate is used in ethereal solution.

In another preferred embodiment, the peroxy compound is selected from the group consisting of hydrogen peroxide, peracetic acid, m-chloroperbenzoic acid and *tert*-butyl hydroperoxide.

In yet another preferred embodiment, the alkali and/or earth alkali hydroxide in the reation with the peroxy compound is selected from the group consisting of LiOH, NaOH, KOH, Ca(OH)₂ and Mg(OH)₂.

In a further preferred embodiment, the base of the alkylation reaction is an alkali and/or earth alkali hydroxide, selected from the group consisting of LiOH, NaOH, KOH, Ca(OH)₂ and Mg(OH)₂.

In another preferred embodiment the alkylating agent is a C₁₋₆-alkyl halide, a C₅₋₁₀-cycloalkyl halide or dimethyl sulfate. Preferably the C₁₋₆-alkyl halide is a C₁₋₆-alkyl bromide or C₁₋₆-alkyl iodide. Particularly preferred the alkylating agent is iodomethane or dimethyl sulfate.

In a particularly preferred embodiment, the metal-iodine exchange of the second reaction sequence is carried out with at least one mol of elementary iodine per equivalent of metal to be exchanged.

Preferably the halogen-metal exchange of the second reaction sequence is carried out with one equivalent of *n*-butyllithium at a temperature below -40 °C in a polar solvent. The following metal-iodine exchange is carried out with at least one mol of elementary iodine per equivalent of metal to be exchanged. Particularly preferred the second reaction sequence is carried out at a temperature below -70 °C in tetrahydrofuran.

In a preferred process, wherein a further reaction sequence is carried out starting with the compound of formula V above, comprising a low temperature halogen-metal exchange of one iodine atom of the aryl moiety containing two halogen atoms and subsequent metal-phosphine exchange, to afford a compound of formula 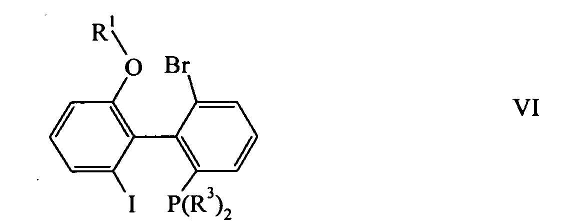 wherein R¹ is as defined above, and
R³ is selected from the group consisting of aryl, C₅₋₁₀-cycloalkyl and C₁₋₆-alkyl, wherein each aryl moiety is optionally substituted with one or more substituents selected from the group consisting of halogen atoms, nitro, amino, C₁₋₆-alkyl, C₁₋₆-alkoxy and di-C₁₋₆-alkylamino groups, and each C₁₋₆-alkyl, C₁₋₆-alkoxy, di-C₁₋₆-alkylamino and C₅₋₁₀-cycloalkyl group in R³ optionally being substituted with one or more halogen atoms,
and the remaining iodine atom of the compound of formula VI is replaced in a low temperature halogen-metal exchange and subsequent metal-phosphine exchange, to afford a compound of formula 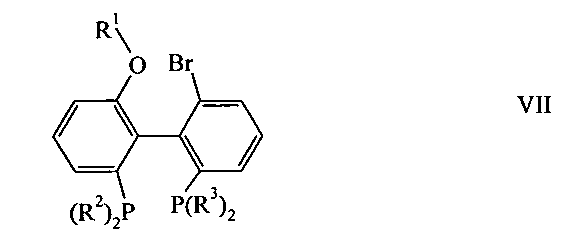 wherein R¹ and R³ are as defined above, and
R² and R³ are independently selected from the group consisting of aryl, C₅₋₁₀-cycloalkyl and C₁₋₆-alkyl, wherein each aryl moiety is optionally substituted with one or more substituents selected from the group consisting of halogen atoms, nitro, amino, C₁₋₆-alkyl, C₁₋₆-alkoxy and di-C₁₋₆-alkylamino groups, and each C₁₋₆-alkyl, C₁₋₆-alkoxy, di-C₁₋₆-alkyl amino and C₅₋₁₀-cycloalkyl group in R² and R³ optionally being substituted with one or more halogen atoms,
and the remaining bromine atom of the compound of formula VII is replaced with hydrogen by high temperature halogen-metal exchange and subsequent reaction with a proton donor, to afford ligands of formula 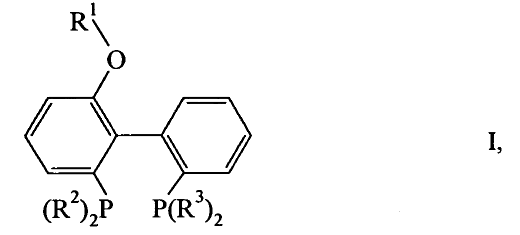 wherein R¹, R² and R³ are as defined in compound VII above.

In a preferred embodiment R² and R³ are the same. In this case, the halogen-metal and the metal-phosphine exchange sequences each can be carried out, using two equivalents of the respective agents. This avoids isolation of the compunds of formula VI and is depicted in Scheme 2 with the shortcut from formula V to formula VII.

In a further preferred embodiment, each low temperature halogen-metal exchange is carried out with an organometallic compound such as *n*-butyllithium, *tert*-butyllithium, isopropylmagnesium chloride or lithium tributylmagnesate at a temperature below -40 °C, preferably in the range of -60 to -90 °C.

In a preferred embodiment each low temperature halogen-metal exchange is carried out in a polar solvent, preferably containing tetrahydrofuran.

The removal of the last remaining bromine atom from compounds of formula VII, as depicted in Scheme 2, requires different reaction conditions for the halogen-metal exchange. In this reaction sequence, in a preferred embodiment, the halogen-metal exchange is carried out with an organometallic compound such as *n*-butyllithium, isopropylmagnesium chloride or lithium tributylmagnesate, at a temperature of at least 0 °C, preferably in the range of 0 to +40 °C. The amount of the organometallic compound (1 to 2 equivalents) depends on the substituents attached to the biaryl moiety. In most cases one equivalent of the organometallic compound is sufficient to replace the halogen atom with the metal.
In a preferred embodiment the high temperature halogen-metal exchange is carried out in a solution containing toluene and/or tetrahydrofuran.

In a preferred embodiment the metal-phosphine exchange is carried out using a halophosphine of the formula 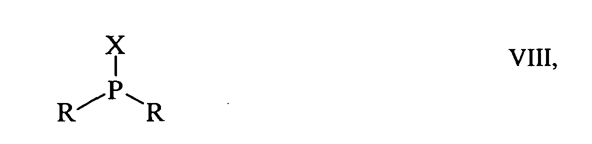 wherein X is chlorine, bromine or iodine and both substituents R are equal and are R² or R³, wherein R² and R³ are as defined above.

Depending on the intended substituents, said halophosphine of the formula VIII is selected from the group consisting of halodiarylphosphines, halodi-(C₅₋₁₀-cycloalkyl)phosphines and halodi-(C₁₋₆-alkyl)phosphines.

Each aryl moiety of the halodiarylphosphine is optionally substituted with one or more substituents selected from the group consisting of halogen atoms, nitro, amino, C₁₋₆-alkyl, C₁₋₆-alkoxy and di-C₁₋₆-alkylamino groups. Optionally each C₁₋₆-alkyl, C₁₋₆-alkoxy, di-C₁₋₆-alkylamino and C₅₋₁₀-cycloalkyl group of the halophosphine of the formula VIII is substituted with one or more halogen atoms. In a preferred embodiment the halophosphine of the formula VIII is selected from the group consisting of halodiarylphosphines and halodi-(C₅₋₁₀-cycloalkyl)phosphines, more preferably is chlorodicyclohexylphosphine, bromodicyclohexylphosphine, chlorodiphenylphosphine or bromodiphenylphosphine.

In a preferred embodiment the hydrogen donor is selected from the group consisting of C₁₋₃-alcohols, water, non-oxidizing inorganic proton acids, and C₁₋₃-alkanoic acids. Preferably the non-oxidizing inorganic proton acid is HCl.

Preferably, the reaction with the hydrogen donor (hydrogen quenching) is carried out at a temperature in the range of -60 to -90 °C.

Provided are compounds of formula 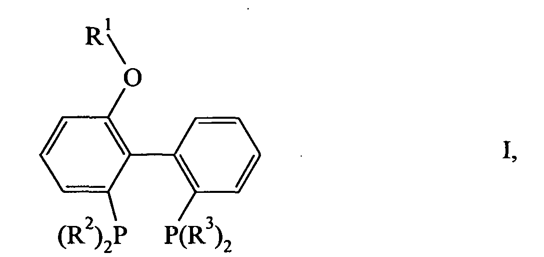 wherein R¹ is C₁₋₆-alkyl or C₃₋₁₀-cycloalkyl optionally substituted with one or more halogen atoms, and
R² and R³ are independently selected from the group consisting of aryl, C₅₋₁₀-cycloalkyl and C₁₋₆-alkyl, wherein each aryl moiety is optionally substituted with one or more substituents selected from the group consisting of halogen atoms, nitro, amino, C₁₋₆-alkyl, C₁₋₆-alkoxy and di-C₁₋₆-alkylamino groups, and each C₁₋₆-alkyl, C₁₋₆-alkoxy, di-C₁₋₆-alkylamino and C₅₋₁₀-cycloalkyl group in R² and R³ optionally being substituted with one or more halogen atoms.

Furthermore provided are compounds of formula 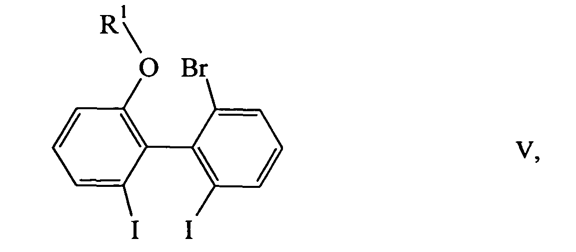 wherein R¹ is C₁₋₆-alkyl or C₃₋₁₀-cycloalkyl optionally substituted with one or more halogen atoms.

Provided are compounds of formula 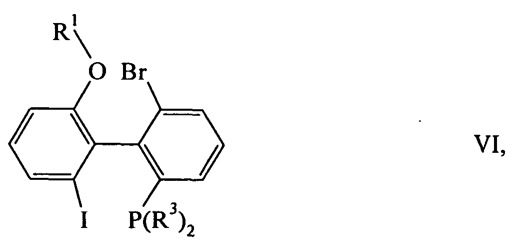 wherein R¹ is C₁₋₆-alkyl or C₃₋₁₀-cycloalkyl optionally substituted with one or more halogen atoms,
and R³ is selected from the group consisting of aryl, C₅₋₁₀-cycloalkyl and C₁₋₆-alkyl, wherein each aryl moiety is optionally substituted with one or more substituents selected from the group consisting of halogen atoms, nitro, amino, C₁₋₆-alkyl, C₁₋₆-alkoxy and di-C₁₋₆-alkylamino groups, and each C₁₋₆-alkyl, C₁₋₆-alkoxy, di-C₁₋₆-alkylamino and C₅₋₁₀-cycloalkyl group in R³ optionally being substituted with one or more halogen atoms.

Furthermore provided are compounds of formula 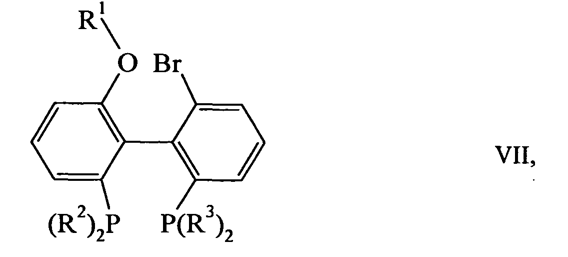 wherein R¹ is C₁₋₆-alkyl or C₃₋₁₀-cycloalkyl optionally substituted with one or more halogen atoms, and
R² and R³ are independently selected from the group consisting of aryl, C₅₋₁₀-cycloalkyl and C₁₋₆-alkyl, wherein each aryl moiety is optionally substituted with one or more substituents selected from the group consisting of halogen atoms, nitro, amino, C₁₋₆-alkyl, C₁₋₆-alkoxy and di-C₁₋₆-alkylamino groups, and each C₁₋₆-alkyl, C₁₋₆-alkoxy, di-C₁₋₆-alkylamino and C₅₋₁₀-cycloalkyl group in R² and R³ optionally being substituted with one or more halogen atoms.

Provided is the use of compounds of formula 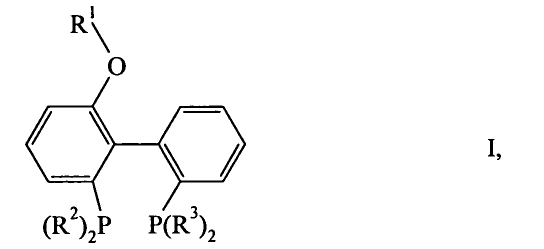 wherein R¹ is C₁₋₆-alkyl or C₃₋₁₀-cycloalkyl optionally being substituted with one or more halogen atoms,
R² and R³ are independently selected from the group consisting of aryl, C₅₋₁₀-cycloalkyl and C₁₋₆-alkyl, wherein each aryl moiety is optionally substituted with one or more substituents selected from the group consisting of halogen atoms, nitro, amino, C₁₋₆-alkyl, C₁₋₆-alkoxy and di-C₁₋₆-alkylamino groups, and each C₁₋₆-alkyl, C₁₋₆-alkoxy, di-C₁₋₆-alkylamino and C₅₋₁₀-cycloalkyl group in R² and R³ optionally being substituted with one or more halogen atoms,
for the preparation of catalytic active complexes of transition metals, preferably of ruthenium, rhodium or iridium. Said catalytic active complexes of transition metals can be used for hydrogenating, preferably asymmetrically hydrogenating, of a compound containing at least one unsaturated prochiral system. Preferably the products obtained by said asymmetrically hydrogenating are enantiomerically pure compounds.

Several examples for general applicable methods for the preparations of catalysts and catalyst solutions are disclosed in Ashworth, T. V. et al. *S. Afr. J. Chem*. **1987,** *40*, 183-188, WO 00/29370 and Mashima, K. *J. Org. Chem*. **1994,** *59*, 3064-3076.

In a preferred embodiment the hydrogen pressure during above mentioned hydrogenating is in the range of 1 to 60 bar, particularly preferred in the range of 2 to 35 bar.
In a further preferred embodiment hydrogenating is carried out at a temperature in the range of 0 to 150 °C.

In a preferred embodiment, the compounds containing at least one unsaturated prochiral system are selected from the group consisting of compounds containing a prochiral carbonyl group, a prochiral alkene group or a prochiral imine group.

In a particular preferred embodiment, the compound containing at least one unsaturated prochiral carbonyl, alkene or imine group is selected from the group consisting of α- and β-ketoesters, α- and β-ketoamines, α- and β-ketoalcohols, acrylic acid derivatives, acylated enamines or N-substituted imines of aromatic ketones and aldehydes.
Preferably the hydrogenation reactions are carried out with a catalyst solution in a polar solvent like C₁₋₄-alcohols, water, dimethyl sulfoxide (DMSO), dimethylformamide (DMF), acetonitrile (MeCN), ethers or mixtures thereof. Preferably the polar solvent contains methanol, ethanol or isopropyl alcohol or a mixture thereof. Particularly preferred, the solution may contain further additives.

The present invention is illustrated by the following non-limiting examples.

### Examples:

### Example 1: 1,3-Dibromo-2-iodobenzene (II)

Diisopropylamine (0.14 L, 0.10 kg, 1.0 mol) and 1,3-dibromobenzene (0.12 L, 0.24 kg, 1.0 mol) were consecutively added to a solution of *n*-butyllithium (1.0 mol) in tetrahydrofuran (2.0 L) and hexanes (0.64 L) at -75 °C. After 2 h at -75 °C, a solution of iodine (0.26 kg, 1.0 mol) in tetrahydrofuran (0.5 L) was added. The solvents were evaporated and the residue dissolved in diethyl ether (1.0 L). After washing with a 10% aqueous solution of sodium thiosulfate (2×0.1 L), the organic layer was dried over sodium sulfate before being evaporated to dryness. Upon crystallization from ethanol (1.0 L), colorless platelets were obtained;
m.p. 99 to 100 °C; 0.33 kg (91%);
¹H-NMR (CHCl₃, 400 MHz): δ = 7.55 (d, *J*= 8.1 Hz, 2 H), 7.07 (t, J = 8.1 Hz, 2 H); C₆H₃Br₂I (361.80): calculated (%) C 19.92, H 0.84; found C 19.97, H 0.80.

### Example 2: 2,2',6,6'-Tetrabromo-1,1'-biphenyl (III)

At -75 °C butyllithium (14 mmol) in hexanes (5.6 mL) was added to a solution of 1,3-dibromo-2-iodobenzene (4.3 g, 12 mmol) in diethyl ether (0.18 L). After the solution was stirred for 2 h at -75 °C, copper(II)chloride (9.7 g, 72 mmol) was added, and the reaction mixture was allowed to attain 25 °C over a 12 h period. Cold water was added to the reaction mixture and the organic layer was separated. The aqueous phase was extracted with ethyl acetate (2×0.10 L). The combined organic layers were dried over sodium sulfate
before being evaporated. 2,2',6,6'-tetrabromo-1,1'-biphenyl precipitates upon treatment of the residue with hexanes cooled to -20 °C. The product is pure enough for further reaction; m.p. 214 - 215 °C; 9.0 g (33%);
¹H NMR (CDCl₃, 400 MHz): δ = 7.67 (d, *J*= 8.3 Hz, 4 H), 7.17 (t, *J* = 8.0 Hz, 2 H).

### Example 3: 2,6,6'-Tribromo-2'-methoxy-1,1'-biphenyl (IVa; R¹ = Me)

*n*-Butyllithium (0.10 mol) in hexanes (63 mL) was added at -75 °C to a solution of 2,2',6,6'-tetrabromo-1,1'-biphenyl (47 g, 0.10 mol) in tetrahydrofuran (0.50 L). The mixture was consecutively treated with fluorodimethoxyborane diethyl ether adduct (19 mL, 16 g, 0.10 mol), a 3.0 M aqueous solution of sodium hydroxide (36 mL) and 30% aqueous hydrogen peroxide (10 mL, 3.6 g, 0.10 mol). The reaction mixture was neutralized at 25 °C with 2.0 M hydrochloric acid (0.10 L) and extracted with diethyl ether (3 x0.10 L). The combined organic layers were washed with a 10% aqueous solution of sodium sulfite (0.10 L), dried over sodium sulfate and evaporated. The oily residue was dissolved in dimethyl sulfoxide (0.20 L) before iodomethane (7.5 mL, 17 g, 0.12 mol) and potassium hydroxide powder (6.7 g, 0.12 mol) were consecutively added. After 1 h, water (0.50 L) was added and the product was extracted with diethyl ether (3×0.10 L). The organic layers were dried over sodium sulfate and evaporated. Crystallization form ethanol (0.10 L) afforded 35 g (82%) product as colorless cubes;
m.p. 184 to 185 °C;
¹H-NMR (CDCl₃, 400 MHz): δ = 7.64 (d, *J*= 8.3 Hz, 2 H), 7.3 (m, 2 H), 7.11 (t, *J*= 8.1 Hz, 1 H), 6.96 (dd, *J* = 7.2, 2.2 Hz, 1 H), 3.77 (s, 3 H);
C₁₃H₉Br₃ (420.92): calculated (%) C 37.09, H 2.16; found C 37.10, H 2.03.

### Example 4: 2-Bromo-2',6-diiodo-6'-methoxy-1,1'-biphenyl (Va; R¹ = Me)

*n*-Butyllithium (30 mmol) in hexanes (19 mL) was added at -75 °C to a solution of 2,6,6'-tribromo-2'-methoxy-1,1'-biphenyl (6.3 g, 15 mmol) in tetrahydrofuran (75 mL). 45 Min later, a solution of iodine (7.7 g, 30 mmol) in tetrahydrofuran (20 mL) was added. At 25 °C, a 10% aqueous solution of sodium thiosulfate was added (100 mL) followed by extraction with ethyl acetate (3x50 mL). The organic layer was dried over sodium sulfate before being evaporated to dryness. Crystallization form a 9 : 1 mixture (v/v) hexanes /ethyl acetate (30 mL) afforded colorless needles; 6.0 g (78%).
¹H-NMR (CHCl₃, 300 MHz): δ = 7.90 (*dd, J=* 7.9, 1.1 Hz, 1 H), 7.67 (*dd, J=* 8.0, 1.1 Hz, 1 H), 7.57 (dd, *J =* 7.9, 0.9 Hz, 1 H), 7.13 (t, *J* = 8.1 Hz, 1 H), 6.99 (d, *J =* 8.3 Hz, 1 H), 6.93 (t, *J*= 7.9 Hz, 1 H), 3.78 (s, 3H).

### Example 5: 2-Bromo-6-(dicyclohexylphosphino)-2'-iodo-6'-methoxy-1,1'-biphenyl (VIa; R¹ = Me and R³ = cyclohexyl)

At -75 °C, *n*-butyllithium (10 mmol) in hexanes (6.3 mL) was added to a solution of 2-Bromo-2',6-diiodo-6'-methoxy-1,1'-biphenyl (5.2 g, 10 mmol) in diethyl ether (50 mL). After the addition was completed, the mixture was treated with chlorodicyclohexylphosphine (2.2 mL, 2.3 g, 10 mmol). The mixture was allowed to reach 25 °C and treated with a saturated aqueous solution of ammonium chloride (20 mL). The mixture was extracted with ethyl acetate (3×20 mL), and the combined organic layers were dried over sodium sulfate. After concentration of the solvent, the residue was subjected to purification by silica gel chromatographie (hexane:ethyl acetate; 9:1; v:v) to obtain 4.2 g (72%) of the objective compound as a yellowish oil.
¹H NMR (CDCl₃, 400 MHz): δ = 7.58 (d, *J =* 7.9 Hz, 1 H), 7.5 (m, 2 H), 7.17 (t, *J* = 7.7 Hz, 1 H), 7.01 (t, *J* = 8.1 Hz, 1 H), 6.80 (d, *J* = 8.3 Hz, 1 H), 3.62 (s, 3 H), 1.6 (m, 12 H), 1.1 (m, 10 H);
³¹P-NMR (CDCl₃, 162 MHz): δ = -2.1 (s).

### Example 6: 2-Bromo-6-(dicyclohexylphosphino)-2'-iodo-6'-methoxy-1,1'-biphenyl (VIa; R¹ = Me and R³ = cyclohexyl)

To a solution of butylmagnesium chloride (5.0 mmol) in tetrahydrofuran (13 mL) was added *n*-butyllithium (10 mmol) in hexanes (6.3 mL) at 0 °C and the mixture was stirred for 10 min. A solution of 2-bromo-2',6-diiodo-6'-methoxy-1,1'-biphenyl (5.2 g, 10 mmol) in tetrahydrofuran (20 mL) was added at -75 °C. After stirring for 1 h at -75 °C chlorodicyclohexylphosphine (3.3 mL, 3.5 g, 15 mmol) was added. After stirring at 25 °C for 2 h, the mixture was treated with a saturated aqueous solution of ammonium chloride (20 mL). The mixture was extracted with ethyl acetate (3×20 mL), and the combined organic layers were dried over sodium sulfate. After concentration of the solvent, the residue was subjected to purification by silica gel chromatographie (hexane:ethyl acetate; 9:1; v:v) to obtain 3.6 g (61%) of the objective compound as a yellowish oil.
¹H NMR (CDCl₃, 400 MHz): δ = 7.58 (d, *J* = 7.9 Hz, 1 H), 7.5 (m, 2 H), 7.17 (t, *J* = 7.7 Hz, 1 H), 7.01 (t, *J*= 8.1 Hz, 1 H), 6.80 (d, *J*= 8.3 Hz, 1 H), 3.62 (s, 3 H), 1.6 (m, 12 H), 1.1 (m, 10 H);
³¹P-NMR (CDCl₃, 162 MHz): δ =-2.1 (s).

### Example 7: 2-Bromo-6-(dicyclohexylphosphino)-2'-(diphenylphosphino)-6'-methoxy-1,1'-biphenyl (VIIa; R¹ = Me, R² = phenyl and R³ = cyclohexyl)

At -75 °C, *n*-butyllithium (10 mmol) in hexanes (6.3 mL) was added to a solution of 2-Bromo-6-(dicyclohexylphosphino)-2'-iodo-6'-methoxy-1,1'-biphenyl (5.9 g, 10 mmol) in diethyl ether (20 mL). After 15 min chlorodiphenylphosphine (1.8 mL, 2.2 g, 25 mmol) was added. The mixture was allowed to reach 25 °C. A saturated aqueous solution of ammonium chloride (20 mL) was added and the organic layer was separated. The aqueous phase was extracted with ethyl acetate (3×20 mL) and the combined organic layers were dried over sodium sulfate before being evaporated. The residue was subjected to purification by silica gel chromatographie (cyclohexane as eluent) to obtain 4.1 g (63%) of the objective compound as a yellowish oil.
¹H-NMR (CDCl₃, 400 MHz): δ = 7.46 (d, *J* =7.9 Hz, 1 H), 7.39 (d, *J* = 7.4 Hz, 1 H), 7.33 (dd, *J* = 7.7, 2.4 Hz, 1 H), 7.2 (m, 12 H), 6.82 (d, *J* = 7.4 Hz, 1 H), 3.65 (s, 3 H), 1.6 (m, 12 H), 1.0 (m, 10 H).

### Example 8: 2'-(Dicyclohexylphosphino)-2-(diphenylphosphino)-6-methoxy-1,1'-biphenyl (Id; R¹ = Me, R² = phenyl and R³ = cyclohexyl)

At 0 °C, *n*-butyllithium (4.6 mmol) in hexanes (2.9 mL) was added to a solution of 2-bromo-6-(dicyclohexylphosphino)-2'-(diphenylphosphino)-6'-methoxy-1,1'-biphenyl (2.5 g, 3.9 mmol) in toluene (6.0 mL). After 1 h at 0 °C, methanol (1 mL) was added followed by water (10 mL) and the organic layer was separated. The aqueous phase was extracted with dichloromethane (2×20 mL) and the combined organic layers were dried over sodium sulfate before being evaporated. The residue was subjected to purification by silica gel chromatographie (hexane:ethyl acetate; 9:1;v:v) to obtain 0.94 g (43%) of the objective compound. Crystallization from ethyl acetate (10 mL) afforded colorless prisms.
¹H-NMR (CDCl₃, 400 MHz): δ = 7.50 (d, *J* = 7.8 Hz, 1 H), 7.2 (m, 12 H), 6.98 (t, *J*= 7.5 Hz, 1 H), 8.83 (d, *J* = 8.2 Hz, 1 H), 6.66 (ddd, *J* = 7.7, 3.2, 0.9 Hz, 1 H), 6.57 (ddd, *J* = 7.5, 3.7, 1.2 Hz, 1 H), 3.64 (s, 3 H), 1.6 (m, 12 H), 1.1 (m, 10 H);
³¹P-NMR (CDCl₃, 162 MHz): δ =-8.7 (d, *J* = 13.1 Hz), -13.4 (d, *J* = 13.2 Hz); C₃₇H₄₂OP₂ (564.27): calculated (%) C 78.70, H 7.50; found C 78.42, H 7.65.

### Example 9: 6-Bromo-2,6'-bis(diphenylphosphino)-2'-methoxy-1,1'-biphenyl (VIIb; R¹ = Me and R² = R³ = phenyl)

At -75 °C, *n*-butyllithium (0.10 mol) in hexanes (57 mL) was added to a solution of compound Va (26 g, 50 mmol) in tetrahydrofuran (0.25 L). The reaction mixture was treated with a 2.0 M solution of chlorodiphenylphosphine (18 mL, 22 g, 100 mmol) in tetrahydrofuran (50 mL). The mixture was allowed to reach 25 °C and treated with a saturated aqueous solution of ammonium chloride (0.20 L). The reaction mixture was extracted with ethyl acetate (3×0.10 L), and the combined organic layers were dried over sodium sulfate. Evaporation of the solvents and crystallization from ethyl acetate (0.10 L) afforded colorless needles;
m.p. 224 to 226 °C (decomposition);
¹H-NMR (CDCl₃, 400 MHz): δ = 7.56 (dd, *J =* 8.0, 1.3 Hz, 1 H), 7.38 (dt, *J* = 7.0, 1.6 Hz, 2 H), 7.3 (m, 12 H), 7.1 (m, 6 H), 6.99 (ddd, *J*= 7.7, 2.9, 1.3 Hz, 1 H), 6.8 (m, 3 H), 6.63 (d, *J* = 8.6 Hz, 1 H), 3.01 (s, 3 H);
³¹P-NMR (CDCl₃, 162 MHz): δ =-10.1 (d, *J*= 24.6 Hz), -13.9 (d, *J* = 24.9 Hz); C₃₇H₂₉BrOP₂ (631.49): calculated (%) C 70.37, H 4.63; found C 69.13, H 4.60.

### Example 10 to 12:

Ligands Ib, Ia and Ic have been prepared according to Scheme 2.

### Example 10: 2,2'-bis(dicyclohexylphosphino)-6-methoxy-1,1'-biphenyl (Ib; R¹ = Me, R² = R³ = cyclohexyl)

Colorless cubes; m.p. 220 to 221 °C (decomposition);
¹H-NMR (CDCl₃, 400 MHz): δ = 7.56 (m sym., 1 H), 7.4 (m, 3 H), 7.16 (d, *J*= 7.5 Hz, 1 H), 7.08 (m sym., 1 H), 6.88 (d, *J* = 7.8 Hz, 1 H), 3.66 (s, 3 H), 1.7 (m, 24 H), 1.2 (m, 20 H);
³¹P-NMR (CDCl₃, 162 MHz): δ = -9.9 (d, *J*= 12.1 Hz), -11.5 (d, *J*= 12.2 Hz); C₃₇H₅₄OP₂ (576.79): calculated (%) C 77.05, H 9.44; found C 77.17, H 9.14.

### Example 11: 2',6-Bis(diphenylphosphino)-2-methoxy-1,1'-biphenyl (Ia; R¹ = Me and R² = R³ = phenyl)

Colorless prisms; m.p. 227 to 228 °C (decomposition); ¹H-NMR (CDC13, 400 MHz): δ = 7.3 (m, 18 H), 7.15 (dt, *J* = 6.8, 2.6 Hz, 4 H), 7.06 (dt, *J* = 7.3, 1.7 Hz, 2 H), 6.82 (dd, *J* = 7.6, 4.2 Hz, 1 H), 6.77 (d, *J* = 8.2 Hz, 1 H), 6.66 (dd, *J* = 7.9 Hz, 3.1, 1 H), 3.22 (s, 3 H);
³¹P-NMR (CDCl₃, 162 MHz): δ =-13.1 (d, *J*= 17.8 Hz), -13.6 (d, *J*= 17.8 Hz); C₃₇H₃₀OP₂ (552.59): calculated (%) C 80.42, H 5.47; found C 80.53, H 5.52.

### Example 12: 6-Dicyclohexylphosphanyl-2'-diphenylphosphanyl-2-methoxy-1,1'-biphenyl (Ic; R¹ = Me, R² = cyclohexyl and R³ = phenyl)

m.p. 170 to 171 °C;
¹H-NMR (CDCl₃, 400 MHz): δ = 7.3 (m, 16 H), 6.78 (d, *J* = 7.9 Hz, 1 H), 3.23 (s, 3 H); ³¹P-NMR (CDCl₃, 162 MHz): δ =-11.3 (d, *J* = 10.7 Hz), -14.0 (d, *J* = 10.8 Hz); C₃₇H₄₂OP₂ (564.69): calculated (%) C 78.70, H 7.50; found C 78.59, H 7.43.

### Example 13: (-)- and (+)-6-Dicyclohexylphosphanyl-2'-diphenylphosphino-2-methoxy-1,1'-biphenyl (Ic; R¹ = Me, R² = cyclohexyl and R³ = phenyl)

The racemic diphosphine Ic was separated into its enantiomers by preparative chromatography using a chiral stationary phase. The column used was CHIRALCEL® OD 20 µm, the mobile phase was n-Heptane / EtOH (2000:1; v:v). From 360 mg racemic material 142 mg of (+)-6-dicyclohexylphosphanyl-2'-diphenylphosphanyl-2-methoxy-1,1'-biphenyl and 123 mg of (-)-6-dicyclohexylphosphanyl-2'-diphenylphosphanyl-2-methoxy-1,1'-biphenyl were isolated. The enantiomeric purity of both compound was 100% (measured by HPLC on an analytic CHIRALCEL® OD 10 µm column), the optical rotation of the (-)-isomer is α_{D}²⁴ (*c* = 0.5 in CH₂Cl₂) = -1.4.

### Example 14 to 16:

The chiral Ligands (-)- and (+)-Ib, (-)- and (+)-Ia and (-)- and (+)-Id are prepared by separation of the corresponding racemic diphosphines according to example 13.

### Example 14: (-)- and (+)-2',6-Bisdicyclohexylphosphino-2-methoxy-1,1'-biphenyl (Ib; R¹ = Me, R² = R³ = cyclohexyl)

The enantiomeric purity was 99.2% for the (-)-isomer and 96.9% for the (+)-isomer (measured by HPLC on an analytic CHIRALCEL® OD 10 µm column), the optical rotation of the (+)-isomer is α_{D}²⁴ (*c* = 0.5 in CH₂Cl₂) = 16.4.

### Example 15: (-)- and (+)-2',6-Bis(diphenylphosphino)-2-methoxy-1,1'-biphenyl (Ia; R¹ = Me, R² = R³ = phenyl)

The optical rotation of the (+)-isomer is α_{D}²⁰ (*c* = 0.5 in CHCl₃) = 6.0.

### Example 16: (-)- and (+)-2'-(Dicyclohexylphosphino)-2-(diphenylphosphino)-6-methoxy-1,1'-biphenyl (Id; R¹ = Me, R² = phenyl and R³ = cyclohexyl)

The optical rotation of the (-)-isomer is α_{D}²⁰ (c = 0.5 in CH₂Cl₂) = -34.9.

### Example 17: (S)-Ethyl 3-hydroxybutyrate

In a 150 mL autoclave under argon atmosphere RuCl₃ (5.3 mg, 0.026 mmol), (+)-ligand Ia (14.3 mg, 0.026 mmol) and ethyl acetoacetate (0.65 g, 5.0 mmol) is dissolved in degassed ethanol (30 mL). After flushing the autoclave with argon hydrogenation is carried out during 6 h at 50°C and at 4 bar hydrogen pressure. After cooling to room temperature the reaction solution is directly analyzed by GC for conversion (column: HP-101 25 m / 0.2 mm) and, after derivatization with trifluoroacetic acid anhydride, enantiomeric excess (column: Lipodex-E 25 m / 0.25 mm). Conversion is 99.7% at an ee of 99.0%.

### Example 18: (R)-Ethyl 3-hydroxybutyrate

In a 15 mL autoclave under argon atmosphere RuCl₃ (1.5 mg, 0.007 mmol), (-)-ligand Ib (4.3 mg, 0.007 mmol) and ethyl acetoacetate (0.15 g, 1.1 mmol) is dissolved in degassed ethanol (7 mL). After flushing the autoclave with argon hydrogenation is carried out during 15 h at 50°C and at 4 bar hydrogen pressure. After cooling to room temperature the reaction solution is directly analyzed by GC for conversion (column: HP-101 25 m /0.2 mm) and, after derivatization with trifluoroacetic acid anhydride, enantiomeric excess (column: Lipodex-E 25 m / 0.25 mm). Conversion is 98.3% at an ee of 86.7%.

### Example 19: (R)-Ethyl 4-chloro-3-hydroxybutyrate

In a 150 mL autoclave in an argon atmosphere bis(1-isopropyl-4-methylbenzene)dichloro-ruthenium (7.6 mg, 0.012 mmol), (+)-ligand Ia (14.0 mg, 0.025 mmol) and ethyl 4-chloro-3-oxobutyrate 98.4% (0.83 g, 5.0 mmol) is dissolved in degassed ethanol (30 mL). After flushing the autoclave with argon hydrogenation is carried out during 4 h at 80 °C and at 4 bar hydrogen pressure. After cooling to room temperature the reaction solution is directly analyzed by GC for conversion (column: HP-101 25 m / 0.2 mm) and ee (column: Lipodex-E 25 m / 0.25 mm). Conversion is 99.9% at an ee of 88%.

### Example 20: (S)-Ethyl 4-chloro-3-hydroxybutyrate

In a 150 mL autoclave under argon atmosphere bis(1-isopropyl-4-methylbenzene)dichloro-ruthenium (7.5 mg, 0.012 mmol), (+)-ligand Ic (14.4 mg, 0.025 mmol) and ethyl 4-chloro-3-oxobutyrate (0.83 g, 5.0 mmol) is dissolved in degassed ethanol (30 mL). After flushing the autoclave with argon hydrogenation is carried out during 3 h at 80 °C and at 4 bar hydrogen pressure. After cooling to room temperature the reaction solution is directly analyzed by GC for conversion (column: HP-101 25 m / 0.2 mm) and ee (column: Lipodex-E 25 m / 0.25 mm). Conversion is 100% at an ee of 80%.

### Example 21: N-Acetyl-D-phenylalanine

In a 15 mL autoclave in an argon atmosphere bis(benzene)dichlor-ruthenium (2.6 mg, 0.005 mmol), (+)-ligand Ia (6.0 mg, 0.011 mmol) and *N*-acetylaminocinnamic acid (0.53 g, 2.5 mmol) is dissolved in degassed methanol (5 mL). After flushing the autoclave with argon hydrogenation is carried out during 16 h at 40 °C and at 50 bar hydrogen pressure. After cooling to room temperature the reaction solution is evaporated and the residue analysed by HPLC for conversion (column: Bischoff Kromasil 100 C8) and enantiomeric excess (column: Nucleodex b-PM). Conversion is 100% at an ee of 43%.

### Example 22: N-Acetyl-L-phenylalanine

In a 15 mL autoclave in an argon atmosphere bis(benzene)dichlor-ruthenium (2.6 mg, 0.005 mmol), (-)-ligand Ib (3.2 mg, 0.006 mmol) and 2-(*N*-acetylamino)-cinnamic acid (0.53 g, 2.5 mmol) is dissolved in degassed methanol (5 mL). After flushing the autoclave with argon hydrogenation is carried out during 15 h at 40 °C and at 50 bar hydrogen pressure. After cooling to room temperature the reaction solution is evaporated and the residue analysed by HPLC for conversion (column: Bischoff Kromasil 100 C8) and enantiomeric excess (column: Nucleodex b-PM). Conversion is 34% at an ee of 66%.

### Example 23: (S)-2-Acetylamino-3-phenyl-propionic acid methyl ester

In a 15 mL autoclave in an argon atmosphere bis(1,5-cyclooctadiene)-rhodium(I) tetrafluoroborate (1.9 mg, 0.005 mmol), (+)-ligand Ic (2.8 mg, 0.005 mmol) and methyl 2-(*N*-acetylamino)-cinnamate (0.10 g, 0.5 mmol) is dissolved in degassed methanol (6 mL). After flushing the autoclave with argon hydrogenation is carried out during 15 h at 25 °C and at 2 bar hydrogen pressure. After cooling to room temperature the reaction solution is evaporated and the residue analysed by HPLC for conversion (column: Bischoff Kromasil 100 C8) and by GC for enantiomeric excess (column: Lipodex-E 25 m /0.25 mm). Conversion is 100% at an ee of 93.8%.

### Example 24: (R)-N-Benzyl-1-phenylethylamine

In a 15 mL autoclave in an argon atmosphere bis(1,5-cyclooctadiene)-di(iridium(I)-dichloride) 98% (6.7 mg, 0.010 mmol), (+)-ligand Ia (11.0 mg, 0.020 mmol), benzylamine (5.6 mg, 0.052 mmol) and *N*-benzyl-*N*-(1-phenylethylidene)amine (0.21 g, 1.0 mmol) is dissolved in degassed methanol (5 mL) and stirred for 1h at room temperature. After flushing
the autoclave with argon hydrogenation is carried out during 17 h at 30 °C and at 50 bar hydrogen pressure. The reaction solution is directly analysed by GC for conversion (column: HP-101 25 m / 0.2 mm) and enantiomeric excess (column: Macherey-Nagel, Nucleodex Beta-PM CC200/4). Conversion is 100% at an ee of 29%.

### Example 25: (S)-N-Benzyl-1-phenylethylamine

In a 15 mL autoclave in an argon atmosphere bis(1,5-cyclooctadiene)-di(iridium(I)-dichloride) 98% (6.7 mg, 0.010 mmol), (+)-ligand Ic (5.7 mg, 0.010 mmol), benzylamine (5.6 mg, 0.052 mmol) and *N*-benzyl-*N*-(1-phenylethylidene)amine (0.21 g, 1.0 mmol) is dissolved in degassed methanol (5 mL) and stirred for 1h at room temperature. After flushing the autoclave with argon hydrogenation is carried out during 15 h at 30 °C and at 50 bar hydrogen pressure. The reaction solution is directly analysed by GC for conversion (column: HP-101 25 m / 0.2 mm) and enantiomeric excess (column: Macherey-Nagel, Nucleodex Beta-PM CC200/4). Conversion is 100% at an ee of 10%.

### Example 26: (R)-Dimethyl methylsuccinate

Bis(1,5-cyclooctadiene)-rhodium(I) tetrafluoroborate (2.1 mg, 0.005 mmol) and ligand (+)-Ic (3.1 mg, 0.005 mmol) are dissolved in 5 mL degassed methanol in a 15 mL autoclave under argon atmosphere. Dimethyl itaconate (97%, 0.15 g, 0.9 mmol) is added via syringe. After flushing the autoclave with argon, hydrogenation is carried out during 15 h at 23 °C and at 2 bar hydrogen pressure. The reaction solution is directly analysed by GC for conversion (column: HP-101 25 m / 0.2 mm) and enantiomeric excess (column: Macherey-Nagel, Nucleodex Beta-PM CC200/4). Conversion is 100% at an ee of 30%.

### Example 28: (R)-Dimethyl methylsuccinate

Bis(1,5-cyclooctadiene)-rhodium(I) tetrafluoroborate (2.1 mg, 0.005 mmol) and (-)-ligand Ib (3.2 mg, 0.006 mmol) are dissolved in 5 mL degassed methanol in a 15 mL autoclave under argon atmosphere. Dimethyl itaconate (97%, 0.15 g, 0.9 mmol) is added via syringe. After flushing the autoclave with argon, hydrogenation is carried out during 15 h at 23 °C and at 2 bar hydrogen pressure. The reaction solution is directly analysed by GC for conversion (column: HP-101 25 m / 0.2 mm) and enantiomeric excess (column: Macherey-Nagel, Nucleodex Beta-PM CC200/4). Conversion is 60% at an ee of 24%.

## Claims

1. A process for the preparation of asymmetrically substituted biaryldiphosphine ligands of the formula 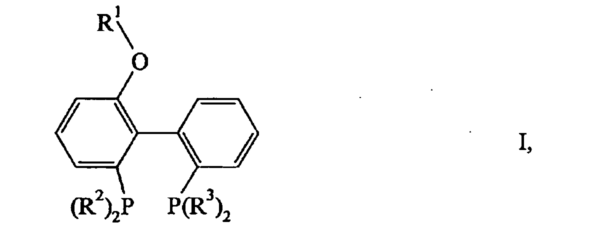 wherein R¹ is C₁₋₆-alkyl or C₃₋₁₀-cycloalkyl optionally substituted with one or more halogen atoms, and
R² and R³ are independently selected from the group consisting of aryl, C₅₋₁₀-cycloalkyl and C₁₋₆-alkyl, wherein each aryl moiety is optionally substituted with one or more substituents selected from the group consisting of halogen atoms, nitro, amino, C₁₋₆-alkyl, C₁₋₆-alkoxy and di-C₁₋₆-alkylamino groups, and each C₁₋₆-alkyl,
C₁₋₆-alkoxy, di-C₁₋₆-alkylamino and C₅₋₁₀cycloalkyl group in R² and R³ optionally being substituted with one or more halogen atoms,
comprising a first reaction sequence, wherein one bromine atom of 2,2',6,6'-tetra-bromobiphenyl 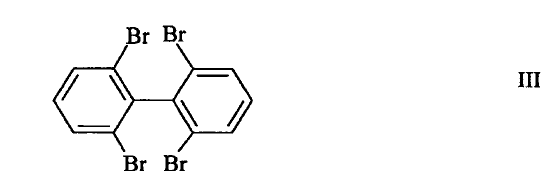 is exchanged with OR¹ by halogen-metal exchange and subsequent metal-hydroxy exchange, followed by an alkylation, to afford a compound of formula 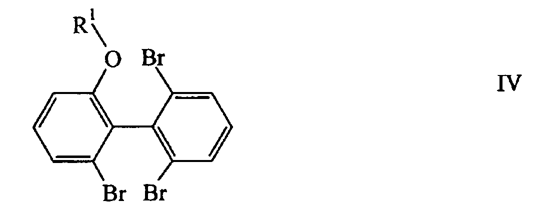 wherein R¹ is as defined above,
and a second reaction sequence, wherein one bromine atom of each aryl moiety of the compound of formula IV is exchanged with iodine by halogen-metal exchange and subsequent metal-iodine exchange, to afford a compound of formula 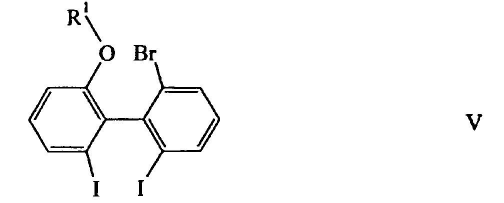 and further reaction sequences, wherein each reaction sequence comprises at least one halogen-metal exchange and subsequent metal-substituent exchange with the respective substituent, thereby exchanging the respective bromine atoms with substituents selected from the group consisting of hydrogen and diarylphosphino, di-C₁₋₆-alkylphosphino and di-C₅₋₁₀-cycloalkylphosphino.

2. The process of claim 1, wherein the first halogen-metal exchange is carried out with one equivalent of *n*-butyllithium at a temperature below -40 °C, the metal-hydroxy exchange is carried out by reacting the metallated intermediate with a borane or organoborate and subsequent reaction with a peroxy compound in the presence of an alkali and/or earth alkali hydroxide, and the alkylation is carried out with an alkylating agent in the presence of a base.

3. The process of any of claim 2, wherein the borane or organoborate is fluoromethoxyborane ethyl ether adduct or trimethylborate.

4. The process of claim 2 or 3, wherein the peroxy compound is selected from the group consisting of hydrogen peroxide, peracetic acid, *m*-chloroperbenzoic acid and *tert*-butyl peroxide.

5. The process of any of claims 2 to 4, wherein the alkylating agent is a C₁₋₆-alkyl halide, a C₅₋₁₀-cycloalkyl halide or dimethyl sulfate.

6. The process of any of claims 2 to 5, wherein the metal-iodine exchange of the second reaction sequence is carried out with at least one mol of elementary iodine per equivalent of metal.

7. The process of any of claims 1 to 6, wherein a further reaction sequence is carried out starting with the compound of formula V, comprising a low temperature halogen-metal exchange of one iodine atom of the aryl moiety containing two halogen atoms and subsequent metal-phosphine exchange, to afford a compound of formula 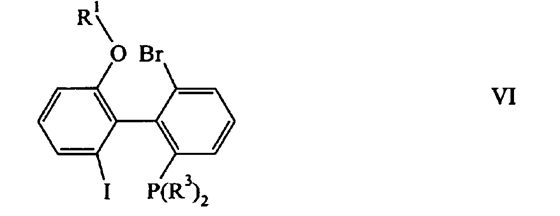 wherein R¹ is as defined above, and
R³ is selected from the group consisting of aryl, C₅₋₁₀-cycloalkyl and C₁₋₆-alkyl, wherein the aryl moiety is optionally substituted with one or more substituents selected from the group consisting of halogen atoms, nitro, amino, C₁₋₆-alkyl, C₁₋₆-alkoxy and di-C₁₋₆-alkylamino groups, and each C₁₋₆-alkyl, C₁₋₆-alkoxy, di-C₁₋₆-alkylamino and C₅₋₁₀-cycloalkyl group in R³ optionally being substituted with one or more halogen atoms,
and the remaining iodine atom of the compound of formula VI is replaced in a low temperature halogen-metal exchange and subsequent metal-phosphine exchange, to afford a compound of formula 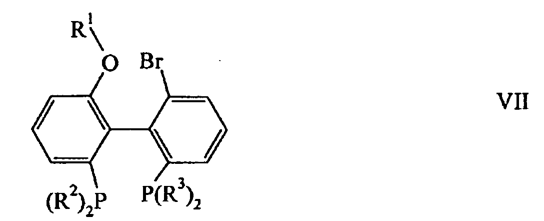 wherein R¹ is as defined above, and
R² and R³ are independently selected from the group consisting of aryl, C₅₋₁₀-cycloalkyl and C₁₋₆-alkyl, wherein each aryl moiety is optionally substituted with one or more substituents selected from the group consisting of halogen atoms, nitro, amino, C₁₋₆-alkyl, C₁₋₆-alkoxy and di-C₁₋₆-alkylamino groups, and each C₁₋₆-alkyl, C₁₋₆-alkoxy, di-C₁₋₆-alkylamino and C₅₋₁₀-cycloalkyl group in R² and R³ optionally being substituted with one or more halogen atoms,
and the remaining bromine atom of the compound of formula VII is replaced with hydrogen by high temperature halogen-metal exchange and subsequent reaction with a proton donor, to afford ligands of formula 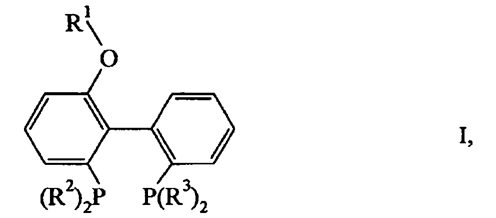 wherein R¹, R² and R³ are as defined in compound VII above.

8. The process of claim 7, wherein the halogen-metal and the metal-phosphine exchange sequences each are carried out using two equivalents of the respective agents.

9. The process of claim 7 or 8, wherein each low temperature halogen-metal exchange is carried out with *n*-butyllithium at a temperature below -40 °C, preferably in the range of -40 to -90 °C.

10. The process of claim 7 or 8, wherein the high temperature halogen-metal exchange is carried out with *n*-butyllithium or *tert-*butyllithium at a temperature of at least +0 °C, preferably in the range of +0 to +40 °C.

11. The process of claim 7 or 8, wherein the metal-phosphine exchange is carried out using a halophosphine of the formula 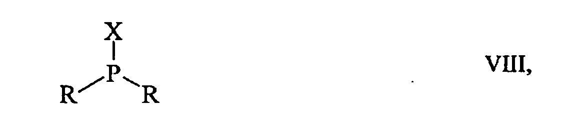 wherein X is chlorine, bromine or iodine and R are equal and are R² or R³, wherein R² and R³ are as defined above.

12. The process of claim 11, wherein the halophosphine of the formula VIII is selected from the group consisting of halodi-(C₅₋₁₀-cycloalkyl)phosphines and halodiarylphosphines, preferably is selected from the group consisting of chlorodicyclohexylphosphine, bromodicyclohexylphosphine, chlorodiphenylphosphine or bromodiphenylphosphine.

13. The process of any of claims 7 to 12, wherein the hydrogen donor is selected from the group consisting of C₁₋₃-alcohols, water, HCl and C₁₋₃-alkanoic acids.

14. Compounds of formula 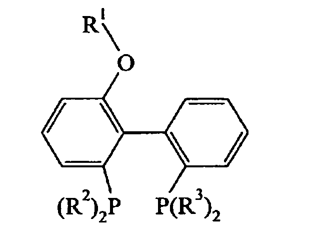 wherein R¹ is C₁₋₆-alkyl or C₃₋₁₀-cycloalkyl optionally substituted with one or more halogen atoms, and
R² and R³ are independently selected from the group consisting of aryl, C₅₋₁₀-cycloalkyl and C₁₋₆-alkyl, wherein each aryl moiety is optionally substituted with one or more substituents selected from the group consisting of halogen atoms, nitro, amino, C₁₋₆-alkyl, C₁₋₆-alkoxy and di-C₁₋₆-alkylamino groups, and each C₁₋₆-alkyl, C₁₋₆-alkoxy, di-C₁₋₆-alkylamino and C₅₋₁₀-cycloalkyl group in R² and R³ optionally being substituted with one or more halogen atoms.

15. Compounds of formula 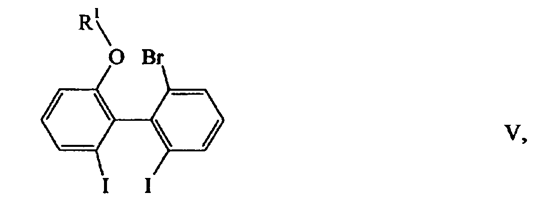 wherein R¹ is C₁₋₆-alkyl or C₃₋₁₀-cycloalkyl optionally substituted with one or more halogen atoms.

16. Compounds of formula 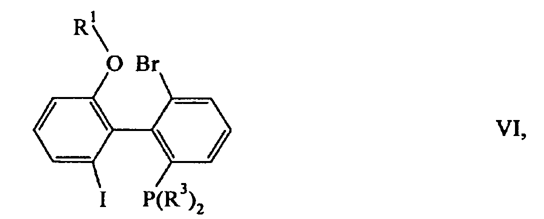 wherein R¹ is C₁₋₆-alkyl or C₃₋₁₀-cycloalkyl optionally being substituted with one or more halogen atoms, and
R³ is selected from the group consisting of aryl, C₅₋₁₀-cycloalkyl and C₁₋₆-alkyl, wherein each aryl moiety is optionally substituted with one or more substituents selected from the group consisting of halogen atoms, nitro, amino, C₁₋₆-alkyl, C₁₋₆-alkoxy and di-C₁₋₆-alkylamino groups, and each C₁₋₆-alkyl, C₁₋₆-alkoxy, di-C₁₋₆-alkylamino and C₅₋₁₀-cycloalkyl group in R³ optionally being substituted with one or more halogen atoms.

17. Compounds of formula 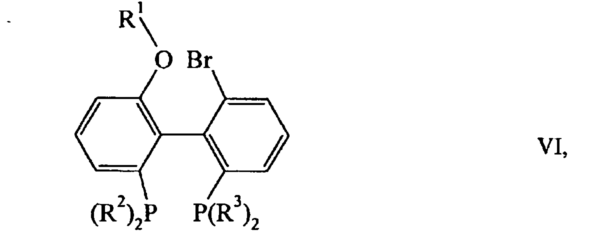 wherein R¹ is C₁₋₆-alkyl or C₃₋₁₀-cycloalkyl optionally being substituted with one or more halogen atoms, and
R² and R³ are independently selected from the group consisting of aryl, C₅₋₁₀-cycloalkyl and C₁₋₆-alkyl, wherein each aryl moiety is optionally substituted with one or more substituents selected from the group consisting of halogen atoms, nitro, amino, C₁₋₆-alkyl, C₁₋₆-alkoxy and di-C₁₋₆-alkylamino groups, and each C₁₋₆-alkyl, C₁₋₆-alkoxy, di-C₁₋₆-alkylamino and C₅₋₁₀-cycloalkyl group in R² and R³ optionally being substituted with one or more halogen atoms.

18. Use of compounds of formula I, 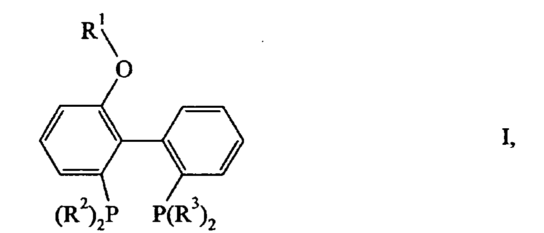 wherein R¹ is C₁₋₆-alkyl or C₃₋₁₀-cycloalkyl optionally substituted with one or more halogen atoms, and
R² and R³ are independently selected from the group consisting of aryl, C₅₋₁₀-cycloalkyl and C₁₋₆-alkyl, wherein each aryl moiety is optionally substituted with one or more substituents selected from the group consisting of halogen atoms, nitro, amino, C₁₋₆-alkyl, C₁₋₆-alkoxy and di-C₁₋₆-alkylamino groups, and each C₁₋₆-alkyl, C₁₋₆-alkoxy, di-C₁₋₆-alkylamino and C₅₋₁₀-cycloalkyl group in R² and R³ optionally being substituted with one or more halogen atoms, for the preparation of catalytic active complexes of transition metals, preferably of ruthenium, rhodium or iridium.
